# EUROPÄISCHE PATENTSCHRIFT

(11) **EP 1 251 818 B1**
(45) Veröffentlichungstag und Bekanntmachung des Hinweises auf die Patenterteilung: **02.07.2008**
(21) Anmeldenummer: 01907448.3
(22) Anmeldetag: 15.01.2001
(51) Int. Cl.: A61K 8/49, A61Q 19/00

(54) **GALENISCHE FORMULIERUNG**
GALENIC FORMULATION
FORMULATION GALENIQUE

(30) Priorität: 28.01.2000 DE 10003786
(43) Veröffentlichungstag der Anmeldung: 30.10.2002
(73) Patentinhaber: Merck Patent GmbH, 64293 Darmstadt (DE)
(72) Erfinder: BÜNGER, Joachim, 64823 Gross-Umstadt (DE); ZUR LAGE, Jutta, 64289 Darmstadt (DE); AXT, Alexandra, 64380 Rossdorf (DE)
(86) Internationale Anmeldenummer: PCT/EP2001/000396
(87) Internationale Veröffentlichungsnummer: WO 2001/054653

(56) Entgegenhaltungen:
- EP-A- 0 521 647
- EP-A- 1 433 477
- DE-A- 19 834 818
- GB-A- 1 308 483
- JP-A- 6 256 175
- JP-A- 53 200 039
- US-A- 5 665 367
- US-A- 5 972 993
- PHOENIX J.; EDWARDS R.H.T.; JACKSON M.J.: 'The effect of vitamin E analogues and long hydrocarbon chain compounds on calcium-induced muscle damage. A novel role for alpha-tocopherol ?' BIOCHIMICA ET BIOPHYSICA ACTA Nr. 1097, 1991, Seiten 212 - 218
- INTERNATONAL COSMETIC INGREDIENT DICTIONARY AND HANDBOOK, Bd. 3, 2004, Seite 1903-1904, 2184-2185,

## Beschreibung

Die Erfindung betrifft stabilisierte kosmetische oder dermatologische Formulierungen enthaltend Hilfs- und/oder Trägerstoffe sowie 2-Hydroxy-5-methyllaurophenonoxim, als Wirkstoff.

Aus der JP 053200039 sind beispielsweise hautaufhellende Kosmetika bekannt, die Vitamin K1 enthalten.

In der EP 0 521 647 werden z.B. kosmetische Zusammensetzungen beschrieben, die Vitamin K1 enthalten und insbesondere zur Anwendung für die Hautbereiche um die Augen geeignet sind.

Die Verwendung von 2-Hydroxy-5-methyllaurophenonoxim, welches auch als HMLO oder F5 bezeichnet wird, in kosmetischen Mitteln ist beispielsweise aus der DE 41 16 123 bekannt. Die darin beschriebenen Mittel sind zur Behandlung von Hauterkrankungen, die mit Entzündungen einhergehen, geeignet. Anwendungsgebiet dieser Mittel sind die pharmazeutische Industrie, die Human- und Veterinärmedizin sowie die Kosmetik. Insbesondere wird beschrieben, daß die Formulierungen enthaltend 2-Hydroxy-5-methyllaurophenonoxim z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können.

Wirkstoffe, die in kosmetische oder dermatologische Formulierungen eingearbeitet werden sollen, sind jedoch oftmals nicht stabil und können in der Formulierung geschädigt werden. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe und/oder ihre Wirksamkeit verlieren.

Bisher ist es beispielsweise nur schwer möglich, Substanzen wie das Vitamin K1 in einer kosmetischen oder dermatologischen Formulierung stabil zu formulieren. Nach dem Einarbeiten von Vitamin K1 in kosmetische oder dermatologische Formulierungen verändern diese Formulierungen z.B. ihre Farbe durch die Einwirkung von UV-Licht und verstärkt durch die Einwirkung von UV-Licht und gleichzeitige Anwesenheit von Luftsauerstoff. Dies führt zu einer heterogenen Braunfärbung der ursprünglich hellen, z.B. hellgelben, kosmetischen oder dermatologischen Formulierung. Derartige Farbveränderungen werden von den Kunden als unästhetisch empfunden.

Aus den obengenannten Gründen ist es notwendig, Wirkstoffe in kosmetischen oder dermatologischen Formulierungen zu stabilisieren.

Es bestand daher die Aufgabe, kosmetische und dermatologische Formulierungen enthaltend Hilfs- und/oder Trägerstoffe sowie 2-Hydroxy-5-methyllaurophenonxim als Wirkstoff zur Verfügung zu stellen, die sich dadurch auszeichnen, daß die Wirkstoffe in der Formulierung eine möglichst hohe Stabilität besitzen und die Formulierungen darüber hinaus auch gegen Verfärbungen stabilisiert sind.

Überraschend wurde nun gefunden, daß diese Aufgabe durch die Bereitstellung von kosmetischen und dermatologischen Formulierungen enthaltend Hilfs- und/oder Trägerstoffe sowie 2-Hydroxy-5-methyllaurophenonoxim, als Wirkstoff gelöst wird, wenn diese zusätzlich
(a) ein oder mehrere Antioxidantien
   und/oder
(b) ein oder mehrere UV-Filter
enthalten.

Die Erfindung betrifft somit kosmetische oder dermatologische Formulierungen enthaltend Hilfs- und/oder Trägerstoffe sowie 2-Hydroxy-5-methyllaurophenonoxim als Wirkstoff dadurch gekennzeichnet, daß sie zusätzlich
(a) ein oder mehrere Antioxidantien
   und/oder
(b) ein oder mehrere UV-Filter
enthalten.

Die Erfindung betrifft weiterhin die Verwendung einer oder mehrerer Verbindungen ausgewählt aus Antioxidantien und UV-Filtern zum Schutz und/oder zur Stabilisierung von in einer kosmetischen oder dermatologischen Formulierung enthaltenen Wirkstoff 2-Hydroxy-5-methyllaurophenonoxim.

Weiterhin betrifft die Erfindung die Verwendung von 2-Hydroxy-5-methyllauro-phenonoxim, und einer oder mehrerer Verbindungen ausgewählt aus Antioxidantien und UV-Filtern zur Herstellung einer kosmetischen oder dermatologischen Formulierung. Die Erfindung betrifft auch die Verwendung des genannten Wirkstoffes, Antioxidantien und UV-Filter zur Herstellung einer kosmetischen oder dermatologischen Formulierung zum Schutz oder zur Pflege der menschlichen oder tierischen Haut.

Desweiteren betrifft die Erfindung ein Verfahren zur Herstellung einer kosmetischen oder dermatologischen Formulierung, dadurch gekennzeichnet, daß man 2-Hydroxy-5-methyllaurophenonoxim, und eine oder mehrere Substanzen ausgewählt aus Antioxidantien und UV-Filtern gegebenenfalls mit Hilfs- und/oder Trägerstoffen in eine geeignete kosmetische oder dermatologische Formulierungsform bringt.

Die in den erfindungsgemäßen kosmetischen oder dermatologischen Formulierungen enthaltenen Antioxidantien und/oder UV-Filter stabilisieren die ebenfalls enthaltenen Wirkstoffe. Dies wirkt sich z.B. in vorteilhafter Weise in einer Erhöhung der Lagerstabilität der erfindungsgemäßen Formulierungen aus.

Die erfindungsgemäßen kosmetischen oder dermatologischen Formulierungen können im Bereich Hautschutz/Hautpflege eingesetzt werden. Sie können zum Schutz oder zur Pflege sowohl der menschlichen als auch der tierischen Haut eingesetzt werden. Der Schutz der menschlichen Haut ist bevorzugt.

In den erfindungsgemäßen Formulierungen können die aus der Fachliteratur bekannten Antioxidantien enthalten sein, z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat) sowie Koniferylbenzoat des Benzoeharzes, Rutinsäure und deren Derivate, α-Glycosylrutin, Ferulasäure, Furfurylidenglucitol, Carnosin, Butylhydroxytoluol (BHT), Butylhydroxyanisol, Nordohydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004),

In einer bevorzugten Ausführungsform der Erfindung enthält die erfindungsgemäße kosmetische oder dermatologische Formulierung als Antioxidans Butylhydroxytoluol.

In einer weiteren bevorzugten Ausführungsform enthält die erfindungsgemäße kosmetische oder dermatologische Formulierung als Antioxidans eine oder mehrere Verbindungen ausgewählt aus Flavonoiden und/oder Coumaranonen.

Als Flavonoide werden die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch dessen Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavanone sind durch folgende Grundstruktur gekennzeichnet:

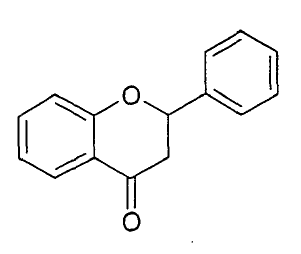

Die Flavone sind durch folgende Grundstruktur gekennzeichnet:

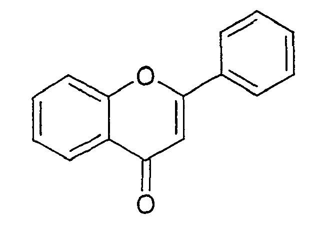

Die 3-Hydroxyflavone (Flavonole) sind durch folgende Grundstruktur gekennzeichnet:

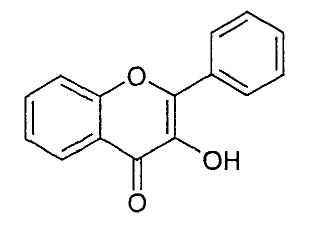

Die Isoflavone sind durch folgende Grundstruktur gekennzeichnet:

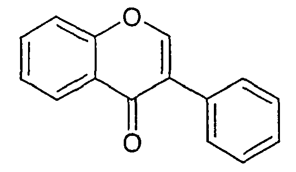

Die Aurone sind durch folgende Grundstruktur gekennzeichnet:

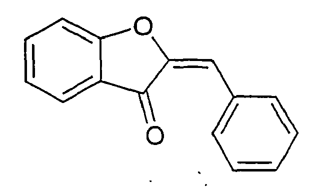

Die Coumaranone sind durch folgende Grundstruktur gekennzeichnet:

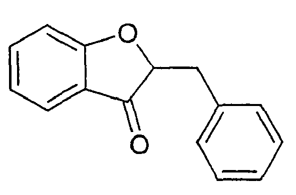

Vorzugsweise sind die Flavonoide und Coumaranone ausgewählt aus den Verbindungen der Formel (I): 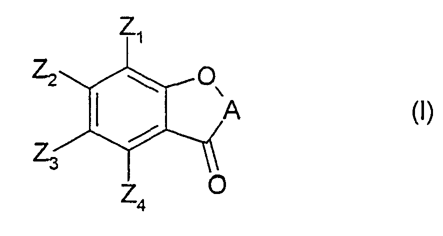 worin
- Z₁ bis Z₄: jeweils unabhängig voneinander H, OH, Alkoxy, Hydroxyalkoxy, Mono- oder Oligoglycosidreste bedeuten und wobei die Alkoxy- und Hydroxyalkoxygruppen verzweigt und unverzweigt sein und 1 bis 18 C-Atome aufweisen können und wobei an die Hydroxy-gruppen der genannten Reste auch Sulfat oder Phosphat gebunden sein kann,
- A: ausgewählt ist aus der Gruppe bestehend aus den Teilformeln (IA), (IB) und (IC) 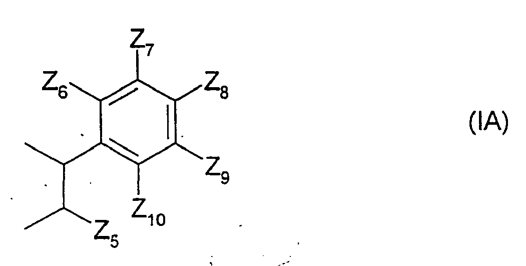 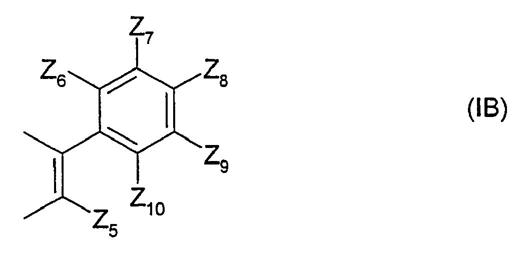 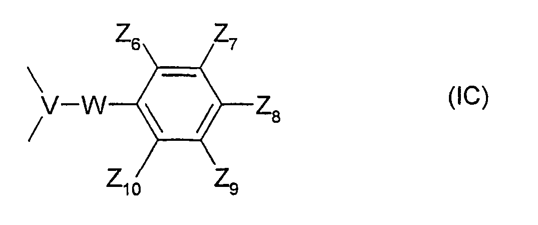
- Z₅: H, OH oder OR,
- R: einen Mono- oder Oligoglykosidrest,
- Z₆ bis Z₁₀: die Bedeutung der Reste Z₁ bis Z₄ besitzen, und 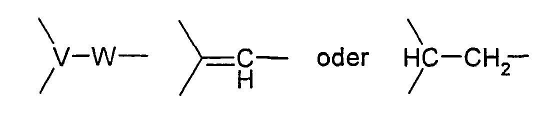
bedeutet.

Die Alkoxygruppen sind vorzugsweise linear und besitzen 1 bis 12 und vorzugsweise 1 bis 8 C-Atome. Diese Gruppen entsprechen somit den Formeln -O-(CH₂)ₘ-H, wobei m 1, 2, 3, 4, 5, 6, 7 oder 8 und insbesondere 1 bis 5 bedeutet.

Die Hydroxyalkoxygruppen sind vorzugsweise linear und besitzen 2 bis 12 und vorzugsweise 2 bis 8 C-Atome. Diese Gruppen entsprechen somit den Formeln -O-(CH₂)ₙ-OH, wobei n 2, 3, 4, 5, 6, 7 oder 8, insbesondere 2 bis 5 und außerordentlich bevorzugt 2 bedeutet.

Die Mono- und Oligoglycosidreste sind vorzugsweise aus 1 bis 3 Glycosideinheiten aufgebaut. Vorzugsweise sind diese Einheiten ausgewählt aus der Gruppe der Hexosylreste insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

In einer bevorzugten Ausführungsform besitzen
- Z₁ und Z₃ die Bedeutung H,
- Z₂ und Z₄ eine andere Bedeutung als H, insbesondere bedeuten sie OH, Methoxy, Ethoxy oder 2-Hydroxyethoxy,
- Z₅ die Bedeutung H, OH oder einen Glycosidrest, der aus 1 bis 3, vorzugsweise aus 1 oder 2, Glycosideinheiten aufgebaut ist,
- Z₆, Z₉ und Z₁₀ die Bedeutung H, und
- Z₇ und Z₈ eine andere Bedeutung als H, insbesondere bedeuten sie OH, Methoxy, Ethoxy oder 2-Hydroxyethoxy.

In einer weiteren bevorzugten Ausführungsform, insbesondere wenn die Wasserlöslichkeit der Flavonoide und Coumaranone gesteigert werden soll, ist an die Hydroxygruppen eine Sulfat- oder Phosphatgruppe gebunden. Geeignete Gegenionen sind beispielsweise die Ionen der Alkali- oder Erdalkalimetalle, wobei diese z.B. aus Natrium oder Kalium ausgewählt sind.

In einer weitern bevorzugten Ausführungsform sind die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten.

Unter den Flavonoiden sind insbesondere Rutin und Troxerutin bevorzugt. Ganz außerordentlich bevorzugt ist Troxerutin.

Unter den Coumaranonen ist 4,6,3',4'-Tetrahydroxybenzylcoumaranon-3 bevorzugt.

Als geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, z.B.

Benzylidenkampferderivate wie
- 3-(4'-Methylbenzyliden)-dl-kampfer (z.B. Eusolex^{®} 6300),
- 3-Benzylidenkampfer (z.B. Mexoryl^{®} SD),
- Polymere von N-{(2 und 4)-[(2-oxobom-3-yliden)methyl]benzyl}-acrylamid (z.B. Mexoryl^{®} SW),
- N,N,N-Trimethyl-4-(2-oxoborn-3-ylidenmethyl)anilinium methylsulfat (z.B. Mexoryl^{®} SK) oder
- α-(2-Oxoborn-3-yliden)toluol-4-sulfonsäure (z.B. Mexoryl^{®} SL),

Benzoyl- oder Dibenzoylmethane wie
- 1-(4-tert-Butylphenyl)-3-(4-methoxyphenyl)propan-1,3-dion (z.B. Eusolex^{®} 9020) oder
- 4-Isopropyldibenzoylmethan (z.B. Eusolex^{®} 8020),

Benzophenone wie
- 2-Hydroxy-4-methoxybenzophenon (z.B. Eusolex^{®} 4360) oder
- 2-Hydroxy-4-methoxybenzophenon-5-sulfonsäure und ihr Natriumsalz (z.B. Uvinul^{®} MS-40),

Methoxyzimtsäureester wie
- Methoxyzimtsäureoctylester (z.B. Eusolex^{®} 2292),
- 4-Methoxyzimtsäureisopentylester, z.B. als Gemisch der Isomere (z.B. Neo Heliopan^{®} E 1000),

Salicylatderivate wie
- 2-Ethylhexylsalicylat (z.B. Eusolex^{®} OS),
- 4-lsopropylbenzylsalicylat (z.B. Megasol^{®}) oder
- 3,3,5-Trimethylcyclohexylsalicylat (z.B. Eusolex^{®} HMS),

4-Aminobenzoesäure und Derivate wie
- 4-Aminobenzoesäure,
- 4-(Dimethylamino)benzoesäure-2-ethylhexylester (z.B. Eusolex^{®} 6007),
- ethoxylierter 4-Aminobenzoesäureethylester (z.B. Uvinul^{®} P25),
und weitere Substanzen wie
- 2-Cyano-3,3-diphenylacrylsäure-2-ethylhexylester (z.B. Eusolex^{®} OCR),
- 2-Phenylbenzimidazol-5-sulfonsäure sowie ihre Kalium-, Natrium- und Triethanolaminsalze (z.B. Eusolex^{®} 232),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl^{®} SX) und
- 2,4,6-Trianilino-(p-carbo-2'-ethylhexyl-1'-oxi)-1,3,5-triazin (z.B. Uvinul^{®} T 150).

Diese organischen UV-Filter werden in der Regel in einer Menge von 0,5 bis 10 Gewichtsprozent, vorzugsweise 1 - 8 %, in die erfindungsgemäßen Formulierungen eingearbeitet

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl[und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (z.B. Parsol^{®} SLX),
- 2,2'-Methylen-bis-(6-(2H-benzotriazol-2-yl)-4-(1,1,3,3-tetramethylbutyl)phenol) (z.B. Tinosorb^{®} M),
- 2,2'-(1,4-Phenylen)bis-(1 H-benzimidazol-4,6-disulfonsäure, Mononatriumsalz) (z.B. Neo Heliopan^{®} AP) und
- 2,4-bis-{[4-(2-Ethyl-hexyloxy)-2-hydroxyl]-phenyl}-6-(4-methoxyphenyl)-1,3,5-triazin (z.B. Tinosorb^{®} S).

Diese organischen UV-Filter werden in der Regel in einer Menge von 0,5 bis 20 Gewichtsprozent, vorzugsweise 1 - 15 %, in die erfindungsgemäßen Formulierungen eingearbeitet.

Als anorganische UV-Filter sind solche aus der Gruppe der Titandioxide wie z.B. gecoatetes Titandioxid (z.B. Eusolex^{®} T-2000 oder Eusolex^{®} T-Aqua), Zinkoxide (z.B. Sachtotec^{®}), Eisenoxide oder auch Ceroxide denkbar. Diese anorganischen UV-Filter werden in der Regel in einer Menge von 0,5 bis 20 Gewichtsprozent, vorzugsweise 2 - 10 %, in die erfindungsgemäßen Formulierungen eingearbeitet.

Bevorzugte UV-Filter sind Zinkoxid, Titandioxid, 3-(4'-Methylbenzyliden)-di-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihren Kalium-, Natrium- und Triethanólaminsalzen.

Besonders bevorzugte UV-Filter sind Zinkoxid und Titandioxid.

Unter den Titandioxid enthaltenden erfindungsgemäßen Formulierungen sind diejenigen bevorzugt, die neben Titandioxid zusätzlich einen oder mehrere weitere UV-Filter ausgewählt aus 3-(4'-Methylbenzyliden)-dikampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihren Kalium-, Natrium- und Triethanolaminsalzen enthalten.

Unter diesen Formulierungen sind diejenigen insbesondere bevorzugt, die neben Titandioxid zusätzlich die UV-Filter 2-Hydroxy-4-methoxy-benzophenon und/oder Methoxyzimtsäureoctylester enthalten.

Die Herstellung der erfindungsgemäßen Formulierung erfolgt, indem 2-Hydroxy-5-methyllaurophenonoxim, und ein oder mehrere Substanzen ausgewählt aus Antioxidantien und UV-Filtem gegebenenfalls mit Hilfs- und/oder Trägerstoffen in eine geeignete Formulierungsform gebracht werden. Die Hilfs- und Trägerstoffe stammen aus der Gruppe der Trägermittel, Konservierungsstoffe und anderer üblicher Hilfsstoffe.

Die erfindungsgemäßen kosmetischen oder dermatologischen Formulierungen werden äußerlich angewendet.

Als Anwendungsform der erfindungsgemäßen Formulierungen seien z.B. genannt: Lösungen, Suspensionen, Emulsionen, Pasten, Salben, Gele, Cremes, Lotionen, Puder, Seifen, tensidhaltige Reinigungspräparate, Öle und Sprays. Weitere Anwendungsformen sind z.B. Sticks, Shampoos und Duschbäder. Der Formulierung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer, Filmbildner, Verdickungsmittel, Feuchthaltemittel.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe. Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten. Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

In einer besonders bevorzugten Ausführungsform der Erfindung liegen die kosmetischen oder dermatologischen Formulierungen in Form einer W/O-Emulsion vor.

Der Anteil des Wirkstoffes 2-Hydroxy-5-methyllaurophenonoxim, in der erfindungsgemäßen Formulierung beträgt vorzugsweise von 0,001 bis 20 Gew.%, besonders bevorzugt von 0,1 bis 3 Gew.% bezogen auf die gesamte erfindungsgemäße Formulierung.

Der Anteil des einen oder der mehreren Antioxidantien in der erfindungsgemäßen Formulierung beträgt vorzugsweise von 0,001 bis 5 Gew.%, besonders bevorzugt von 0,01 bis 2 Gew.% bezogen auf die gesamte erfindungsgemäße Formulierung.

Der Anteil des einen oder mehreren UV-Filter in der erfindungsgemäßen Formulierung beträgt vorzugsweise von 0,05 bis 20 Gew.%, besonders bevorzugt von 1 bis 10 Gew.% bezogen auf die gesamte erfindungsgemäße Formulierung.

Die Feststellung der stabilisierenden Wirkung der Antioxidantien und UV-Filter auf die Wirkstoffe in den erfindungsgemäßen Formulierungen kann nach Methoden, die dem Fachmann bekannt sind, erfolgen. Beispielsweise kann eine erfindungsgemäße Formulierung und eine entsprechende Formulierung ohne Antioxidantien und UV-Filter unter gleichen Bedingungen (Licht- und Lufteinfluß) aufbewahrt werden und durch Vergleich der Verfärbung dieser Formulierungen der stabilisierende Einfluß der Antioxidantien und UV-Filter festgestellt werden.

Alle Verbindungen oder Komponenten, die in den erfindungsgemäßen Formulierungen verwendet werden können, sind entweder bekannt und käuflich erwerbbar oder können nach bekannten Methoden synthetisiert werden.

### Referenz-Beispiele

Alle %-Angaben sind Gewichtsprozent.

Die INCI-Namen der in den Beispielen verwendeten Rohstoffe werden im folgenden in der Englischen Originalbezeichnung angegeben. Sie sind wie folgt:

| | |
|---|---|
| Rohstoff | INCI |
| Antaron V-220 | PVP/Eicosene Glycol |
| Arlacel 165V | Glyceryl Stearate (and) PEG-100 Stearate |
| Arlacel 1689 | Sorbitol, Glycerol Esters |
| BHT | BHT |
| Bienenwachs | Beeswax |
| Cetiol | Oleyl Oleate |
| Cetiol V | Decyl Oleate |
| Cetylalkohol | Cetyl Alcohol |
| Cutina HR | Hydrogenated Castor Oil |
| Emulgator E2155 | Stearyl Alcohol (and) Steareth-7 (and) Steareth-10 |
| Eusolex 2292 | Octyl Methoxycinnamate |
| Eusolex 4360 | Benzophenone-3 |
| Eusolex 6300 | 4-Methylbenzylidene Campher |
| Eusolex 9020 | Butyl Methoxydibenzoylmethane |
| EusolexT-2000 | Titanium Dioxide, Alumina; Simethicone |
| Eusolex T-AQUA | Aqua, Titanium Dioxide, Alumina, Sodium Methaphosphate, Phenoxyethanol, Sodium Methylparaben |
| Euxyl K100 | Benzyl Alcohol, Methylchloroisothiazoline, Methylisothiazoline |
| Germaben II-E | Propylene Glycol, Diazolidinyl Urea, Methylparabene, Propylparabene |
| Glycerin | Glycerin |
| Glycerol | Glycerin |
| Imwitor 900 | Glyceryl Stearate |
| Isolan PDI | Diisosteaoryl Polyglyceryl-3-Diisostearate |
| Isopropylmyristat | Isopropyl Myristate |
| Isopropylpalmitat | Isopropyl Palmitate |
| Karion F flüssig | Sorbitol |
| Lanette 18 | Stearyl Alcohol |
| Lanette O | Cetearyl Alcohol |
| Lanolin | Lanolin |
| Lunacera M | Microwax |
| Luvitol EHO | Cetearyl Octanoate |
| Magnesiumsulfat | Magnesium Sulfate |
| Miglyol 812 | Caprylic/Capric Triglyceride |
| Miglyol 812 Neutralöl | Caprylic/Capric Triglyceride |
| Mirasil CM | Cyclomethicone |
| Mirasil DM 350 | Dimethicone |
| Oxynex K flüssig | PEG-8, Tocopherol, Ascorbyl Palmitate, Ascorbic Acid, Citric Acid |
| Paraffin | Mineral Oil |
| Paraffin, liquid | Mineral Oil |
| Propandiol-1,2 | Propylene Glycol |
| Rutin | Rutin |
| Tegin M | Glyceryl Stearate |
| Teginacid H | Glyceryl Stearate (and) Ceteth-20 |
| Tego Care 450 | Polyglyceryl-3 Methylglucose Distearate |
| Titriplex III | Disodium EDTA |
| Trihydroxyethylrutin | Troxerutin |
| Vitamin K1 | Phytonadione |

### Beispiele 1-3:

### BHT als Antioxidans

### Beispiel 1

Aus folgenden Komponenten werden erfindungsgemäße O/W-Emulsionen enthaltend Vitamin K1 hergestellt:

| | | | Gew.-% |
|---|---|---|---|
| A Paraffin | (Art.-Nr. 107160) | (1) | 8,0 |
| Isopropylmyristat | (Art.-Nr. 822102) | (1) | 7,0 |
| Lanette O | | (2) | 3,0 |
| Arlacel 165V | | (3) | 5,0 |
| | | | |
| BHT | | (4) | x |
| | | | |
| B Wasser, demineralisiert | | | ad 100 |
| Glycerol, 87% | (Art.-Nr. 104091) | (1) | 3,0 |
| Konservierungsmittel | | | q.s. |
| | | | |
| C Vitamin K1 | (Art.-Nr. 501890) | (1) | 1,0 |

In Beispiel 1A ist x = 0,05 und in Beispiel 1 B ist x = 0,1.

Als Konservierungsmittel können
0,05 % Propyl-4-hydroxybenzoat (Art.-Nr. 130173) oder
0,15 % Methyl-4-hydroxybenzoat (Art.-Nr. 130174)
verwendet werden.

### Herstellung:

Die Phasen A und B werden separat auf 75 °C erwärmt und anschließend Phase A unter Rühren in die Phase B eingetragen. Die vereinigten Phasen werden homogenisiert. Danach wird unter Rühren abgekühlt und bei 30 °C Phase C zugesetzt.

### Eigenschaften:

Die Emulsionen sind schwach gelb gefärbt und besitzen einen pH-Wert von 5,7.

### Bezugsquellen:

(1) Merck KGaA
(2) Henkel KGaA
(3) Uniqema
(4) Rhodia

Die Emulsionen der Beispiele 1A und 1 B werden einen Monat an der Luft unter Tageslicht gelagert. Als Vergleich dient eine analoge O/W-Emulsion, die jedoch kein BHT enthält. Die Cremes der Beispiele 1A und 1B zeigen nach dieser Zeit eine deutlich geringere Verfärbung als die Emulsion ohne BHT. Die Emulsionen der Beispiele 1A und 1 B zeigen keinen Unterschied in der Verfärbung.

### Beispiel 2

Aus folgenden Komponenten wird eine erfindungsgemäße W/O-Emulsion enthaltend Vitamin K1 hergestellt:

| | | | Gew.-% |
|---|---|---|---|
| A Arlacel 1689 | | (3) | 6,0 |
| Paraffin, liquid | (Art.-Nr 107162) | (1) | 17,0 |
| Isopropylmyristat | (Art.-Nr.822102) | (1) | 5,0 |
| | | | |
| BHT | | (4) | 0,05 |
| | | | |
| B Wasser, demineralisiert | | | ad 100 |
| Glycerol, 87% | (Art.-Nr. 104091) | (1) | 4,0 |
| Magnesiumsulfat | (Art.-Nr.105886) | (1) | 0,5 |
| Konservierungsmittel | | | q.s. |
| | | | |
| C Vitamin K1 | (Art.-Nr. 501890) | (1) | 1,0 |

Als Konservierungsmittel können
0,05 % Propyl-4-hydroxybenzoat (Art.-Nr. 130173) oder
0,15 % Methyl-4-hydroxybenzoat (Art.-Nr. 130174)
verwendet werden.

### Herstellung:

Die Phasen A und B werden separat auf 75 °C erwärmt und anschließend Phase B unter Rühren in die Phase A eingetragen. Die vereinigten Phasen werden homogenisiert. Danach wird unter Rühren abgekühlt und bei 30 °C Phase C zugesetzt.

### Eigenschaften:

Die Emulsion ist schwach gelb gefärbt.

### Bezugsquellen:

(1) Merck KGaA
(2) Henkel KGaA
(3) Uniqema
(4) Rhodia

Die Emulsion des Beispiels 2 wird analog denen der Beispiele 1A und 1 B getestet. Die Emulsion des Beispiels 2 zeigt eine signifikant geringere Verfärbung als die Emulsion des Beispiels 1A.

### Beispiel 3

Aus folgenden Komponenten wird eine erfindungsgemäße W/O-Emulsion enthaltend Vitamin K1 hergestellt:

| | | | Gew.-% |
|---|---|---|---|
| A Isolan PDI | | (3) | 3,0 |
| Paraffin, liquid | | (1) | 17,0 |
| Isopropylmyristat | (Art.-Nr. 822102) | (1) | 5,0 |
| Bienenwachs | (Art.-Nr.111544) | (1) | 0,2 |
| Cutina HR | | (2) | 0,3 |
| BHT | | (4) | 0,05 |
| | | | |
| B Wasser, demineralisiert | | | ad 100 |
| Glycerol, 87% | (Art.-Nr. 104091) | (1) | 4,0 |
| Magnesiumsulfat | | (1) | 1,0 |
| Konservierungsmittel | | | q.s. |
| | | | |
| C Vitamin K1 | (Art.-Nr. 501890) | (1) | 1,0 |

Als Konservierungsmittel können
0,05 % Propyl-4-hydroxybenzoat (Art.-Nr. 130173) oder
0,15 % Methyl-4-hydroxybenzoat (Art.-Nr. 130174)
verwendet werden.

### Herstellung:

Die Phasen A und B werden separat auf 75 °C erwärmt und anschließend Phase B unter Rühren in die Phase A eingetragen. Die vereinigten Phasen werden homogenisiert. Danach wird unter Rühren abgekühlt und bei 30 °C Phase C zugesetzt.

### Eigenschaften:

Die Emulsion ist schwach gelb gefärbt.

### Bezugsquellen:

(1) Merck KGaA
(2) Henkel KGaA
(3) Uniqema
(4) Rhodia

Die Emulsion des Beispiels 3 wird analog denen der Beispiele 1A und 1 B getestet. Die Emulsion des Beispiels 3 zeigt eine signifikant geringere Verfärbung als die Emulsion des Beispiels 1A. Die Verfärbung der Emulsion des Beispiels 3 ist geringfügig stärker ausgeprägt als die Verfärbung der Emulsion des Beispiels 2.

### Beispiele 4-5:

### Bioflavonoide als Antioxidantien

### Beispiel 4

Aus folgenden Komponenten werden erfindungsgemäße O/W-Emulsionen enthaltend Vitamin K1 hergestellt:

| | | | Gew.-% |
|---|---|---|---|
| A Paraffin | (Art.-Nr. 107160) | (1) | 8,0 |
| Isopropylmyristat | (Art.-Nr.822102) | (1) | 7,0 |
| Lanette O | | (2) | 3,0 |
| Arlacel 165V | | (3) | 5,0 |
| | | | |
| B Wasser, demineralisiert | | | ad 100 |
| Glycerol, 87% | (Art.-Nr. 104091) | (1) | 3,0 |
| Konservierungsmittel | | | q.s. |
| Trihydroxyethylrutin | (Art.-Nr.509002) | (1) | x |
| | | | |
| C Vitamin K1 | (Art.-Nr. 501890) | (1) | 1,0 |

In Beispiel 4A ist x = 0,05, in Beispiel 4B ist x = 0,1, in Beispiel 4C ist x = 0,5 und in Beispiel 4D ist x = 1,0.

Als Konservierungsmittel können
0,05 % Propyl-4-hydroxybenzoat (Art.-Nr. 130173) oder
0,15 % Methyl-4-hydroxybenzoat (Art.-Nr. 130174)
verwendet werden.

### Herstellung:

Die Phasen A und B werden separat auf 75 °C erwärmt und anschließend Phase A unter Rühren in die Phase B eingetragen. Die vereinigten Phasen werden homogenisiert. Danach wird unter Rühren abgekühlt und bei 30 °C Phase C zugesetzt.

### Bezugsquellen:

(1) Merck KGaA
(2) Henkel KGaA
(3) Uniqema

Die Emulsionen des Beispiels 4 werden analog denen der Beispiele 1A und 1 B getestet. Die Emulsionen des Beispiels 4 zeigen eine signifikant geringere Verfärbung als die Emulsion des Beispiels 1A. Zudem läßt sich feststellen, daß die Verfärbung mit steigender Konzentration an Trihydroxyethylrutin geringer wird und die Emulsionen mit 0,5 Gew.-% und 1,0 Gew.-% Trihydroxyethylrutin am geringsten verfärbt sind. Die Emulsionen der Beispiele 4C und 4D (0,5 Gew.-% und 1,0 Gew.-% Trihydroxyethylrutin) zeigen keine Unterschiede in der Verfärbung.

### Beispiel 5

Aus folgenden Komponenten werden erfindungsgemäße O/W-Emulsionen enthaltend Vitamin K1 hergestellt:

| | | | Gew.-% |
|---|---|---|---|
| A Tego Care 450 | | (5) | 3,0 |
| Tegin M | | (5) | 1,0 |
| Lanette 18 | | (2) | 1,0 |
| Miglyol 812 | | (2) | 7,0 |
| Mirasil DM 350 | | (4) | 0,5 |
| Mirasil CM | | (4) | 2,0 |
| Isopropylpalmitat | | (1) | 5,0 |
| BHT | | (4) | x |
| Rutin | (Art.-Nr. 500017) | (1) | y |
| | | | |
| B Wasser, demineralisiert | | | ad 100 |
| Glycerol, 87% | (Art.-Nr. 104091) | (1) | 3,0 |
| Konservierungsmittel | | | q.s. |
| Trihydroxyethylrutin | (Art.-Nr.509002) | (1) | z |
| | | | |
| C Vitamin K1 | (Att.-Nr. 501890) | (1) | 1,0 |

In Beispiel 5A ist x = 0,05, y= 0 und z = 0;
in Beispiel 5B ist x = 0, y = 0 und z = 0,1;
in Beispiel 5C ist x = 0, y = 0 und z = 0,5;
in Beispiel 5D ist x = 0, y = 0,1 und z = 0 und
in Beispiel 5E ist x = 0, y = 0,5 und z = 0.

Als Konservierungsmittel können
0,05 % Propyl-4-hydroxybenzoat (Art.-Nr. 130173) oder
0,15 % Methyl-4-hydroxybenzoat (Art.-Nr. 130174)
verwendet werden.

### Herstellung:

Die Phasen A und B werden separat auf 70 °C erwärmt und anschließend Phase A unter Rühren in die Phase B eingetragen. Die vereinigten Phasen werden homogenisiert. Danach wird unter Rühren abgekühlt und bei 30 °C Phase C zugesetzt.

### Bezugsquellen:

(1) Merck KGaA
(2) Henkel KGaA
(3) Uniqema
(4) Rhodia
(5) Th. Goldschmidt AG

Die Emulsionen des Beispiels 5 werden analog denen der Beispiele 1A und 1 B getestet. Die Emulsionen der Beispiele 5B und 5C (Trihydroxyethylrutin als Antioxidans) zeigen die geringste Veränderung der Farbe, dann die Emulsionen der Beispiele 5D und 5E (Rutin als Antioxidans) und schließlich zeigt die Emulsion des Beispiels 5A im Vergleich die stärkste Veränderung der Farbe.

### Beispiele 6-10

### Formulierungen enthaltend UV-Filter

### Beispiel 6

Aus folgenden Komponenten wird eine erfindungsgemäße O/W-Emulsion enthaltend Vitamin K1 hergestellt:

| | | | Gew.-% |
|---|---|---|---|
| A Arlacel 165V | | (2) | 10,0 |
| Paraffin flüssig | (Art.-Nr. 1.07162) | (1) | 25,0 |
| Cetylalkohol | (Art.-Nr. 1.00989) | (1) | 2,0 |
| Lanolin | | (3) | 2,0 |
| Oxynex K flüssig | (Art.-Nr. 1.08324) | (1) | 0,05 |
| | | | |
| B Zinkoxid | (Art.-Nr. 1.30148) | (1) | 10,0 |
| Karion F flüssig | (Art.-Nr. 1.02993) | (1) | 3,0 |
| Glycerin | (Art.-Nr. 1.04093) | (1) | 2,0 |
| Titriplex III | (Art.-Nr. 1.08421) | (1) | 0,05 |
| Germaben II-E | | (4) | 0,5 |
| Wasser, demineralisiert | | | ad 100 |
| | | | |
| C Vitamin K1 | | (1) | 1,0 |

### Herstellung:

Die Phase A wird auf 75 °C und Phase B separat auf 80 °C erwärmt und anschließend Phase B unter Rühren langsam in die Phase A eingetragen. Die vereinigten Phasen werden homogenisiert. Danach wird unter Rühren abgekühlt und bei 35 °C Phase C zugesetzt.

### Bezugsquellen:

(1) Merck KGaA
(2) ICI
(3) Henry Lamotte
(4) ISP

Das Zinkoxid wirkt der Verfärbung der Emulsion durch Lagerung unter Tageslicht signifikant entgegen.

### Beispiel 7

Aus folgenden Komponenten werden erfindungsgemäße O/W-Emulsionen enthaltend Vitamin K1 hergestellt:

| | | | Gew.-% |
|---|---|---|---|
| A Eusolex T-2000 | (Art.-Nr. 105373) | (1) | a |
| Eusolex 4360 | (Art.-Nr. 105376) | (1) | b |
| Emulgator E2155 | | (2) | 2,0 |
| Teginacid H | | (2) | 2,0 |
| Luvitol EHO | | (3) | 6,0 |
| Imwitor 900 | | (4) | 3,0 |
| Cetiol | | (5) | 5,0 |
| Lunacera M | | (6) | 1,0 |
| Miglyol 812 Neutralöl | | (4) | c |
| Antaron V-220 | | (7) | 1,0 |
| | | | |
| B Propandiol-1,2 | (Art.-Nr. 107478) | (1) | 4,0 |
| Wasser, demineralisiert | | | ad 100 |
| Germaben II-E | | (7) | 0,5 |
| C Vitamin K1 | | (1) | 1,0 |

In Beispiel 7A ist a = 6,0, b = 3,0 und c = 3,0
In Beispiel 7B ist a = 6,0, b = 0 und c = 6,0
In Beispiel 7C ist a = 0, b = 3,0 und c = 3,0

### Herstellung:

Die Phase A wird auf 75 °C und Phase B separat auf 80 °C erwärmt und anschließend Phase B unter Rühren langsam in die Phase A eingetragen. Die vereinigten Phasen werden homogenisiert. Danach wird unter Rühren abgekühlt und bei 35 °C Phase C zugesetzt.

### Bezugsquellen:

(1) Merck KGaA
(2) Th. Goldschmidt
(3) BASF AG
(4) Hüls Troisdorf AG
(5) Henkel KGaA
(6) H.B. Fuller GmbH
(7) ISP

Im folgenden wird die Verfärbung der Emulsionen nach folgenden Einflüssen qualitativ untersucht. Hierbei bedeutet z.B. "Emulsion 1 < Emulsion 2", daß sich Emulsion 1 in signifikanter Weise weniger stark verfärbt als Emulsion 2. "<<" bedeutet, daß dieser Effekt besonders deutlich ausgeprägt ist.
Lagerung unter Einfluß von Tageslicht (4 Wochen):
   Emulsion 7A < Emulsion 7B < Emulsion 7C
Einwirkung von UV-Licht (15 min.; Gerät 'Suntest CPS', 80 W/m²):
   Emulsion 7A < Emulsion 7C << Emulsion 7B
Lagerung unter Lichtausschluß (4 Wochen):
   Emulsion 7B < Emulsion 7A < Emulsion 7C

Die Kombination der UV-Filter Eusolex T-2000 und Eusolex 4360 wirkt der Verfärbung der Emulsion insgesamt signifikant stärker entgegen als die genannten UV-Filter alleine.

### Beispiel 8

Aus folgenden Komponenten werden erfindungsgemäße O/W-Emulsionen enthaltend Vitamin K1 hergestellt:

| | | | Gew.-% |
|---|---|---|---|
| A Eusolex 2292 | (Art.-Nr. 1.05382) | (1) | a |
| Eusolex 4360 | (Art.-Nr. 1.05376) | (1) | b |
| Emulgator E2155 | | (2) | 3,0 |
| Teginacid H | | (2) | 3,0 |
| Luvitol EHO | | (3) | c |
| Imwitor 900 | | (4) | 3,0 |
| Cetiol | | (5) | 4,0 |
| Lunacera M | | (6) | 1,0 |
| Miglyol 812 Neutralöl | | (4) | 3,0 |
| | | | |
| B Eusolex T-AQUA | | (1) | d |
| Propandiol-1,2 | (Art.-Nr. 1.07478) | (1) | 4,0 |
| Allantoin | | (1) | 0,2 |
| Germaben 11-E | | (7) | 0,5 |
| Wasser, demineralisiert | | | ad 100 |
| | | | |
| C Vitamin K1 | | (1) | 1,0 |

In Beispiel 8A ist a = 7,0, b = 3,0, c = 10,5 und d = 16,7
In Beispiel 8B ist a = 0, b = 0, c = 20,5 und d = 16,7
In Beispiel 8C ist a = 7,0, b = 3,0, c = 13,5 und d = 0

### Herstellung:

Die Phase A wird auf 75 °C und Phase B separat auf 80 °C erwärmt und anschließend Phase B unter Rühren langsam in die Phase A eingetragen. Die vereinigten Phasen werden homogenisiert. Danach wird unter Rühren abgekühlt und bei 35 °C Phase C zugesetzt.

### Bezugsquellen:

(1) Merck KGaA
(2) Th. Goldschmidt
(3) BASF AG
(4) Hüls Troisdorf AG
(5) Henkel KGaA
(6) H.B. Fuller GmbH
(7) ISP

Im folgenden wird die Verfärbung der Emulsionen nach folgenden Einflüssen qualitativ untersucht. Hierbei bedeutet z.B. "Emulsion 1 < Emulsion 2", daß sich Emulsion 1 in signifikanter Weise weniger stark verfärbt als Emulsion 2. "<<" bedeutet, daß dieser Effekt besonders deutlich ausgeprägt ist. "Emulsion 1 ~ Emulsion 2" bedeutet, daß Emulsion 1 nach dem entsprechenden Einfluß in ähnlicher Weise verfärbt ist wie Emulsion 2.
Lagerung unter Einfluß von Tageslicht (4 Wochen):
   Emulsion 8A < Emulsion 8B << Emulsion 8C
Einwirkung von UV-Licht (15 min.; Gerät 'Suntest CPS', 80 W/m²):
   Emulsion 8A << Emulsion 8C < Emulsion 8B
Lagerung unter Lichtausschluß (4 Wochen):
   Emulsion 8A ~ Emulsion 8B << Emulsion 8C

Alle genannten UV-Filter-Kombinationen zeigen eine signifikante Verbesserung der Farbstabilität. Die besten Ergebnisse werden mit Titandioxid erzielt bzw. nach UV-Bestrahlung mit Titandioxid unter gleichzeitiger Anwesenheit von Eusolex 2292 und Eusolex 4360.

### Beispiel 9

Aus folgenden Komponenten wird eine erfindungsgemäße W/O-Emulsion enthaltend Vitamin K1 hergestellt:

| | | | Gew.-% |
|---|---|---|---|
| A Eusolex 2292 | (Art.-Nr. 1.05382) | (1) | 7,0 |
| Eusolex 9020 | (Art.-Nr. 1.05844) | (1) | 2,0 |
| Isolan PDI | | (2) | 3,0 |
| Cetiol V | | (3) | 6,0 |
| Luvitol EHO | | (4) | 6,5 |
| Lunacera M | | (5) | 0,5 |
| | | | |
| B Glycerin (etwa 87%) | (Art.-Nr. 1.04091) | (1) | 3,0 |
| Magnesiumsulfat | | | |
| Heptahydrat | (Art.-Nr. 1.05882) | (1) | 1,0 |
| Germaben II-E | | (6) | 0,5 |
| Wasser, demineralisiert | | | ad 100 |
| | | | |
| C Vitamin K1 | | (1) | 1,0 |

### Herstellung:

Die Phase A wird auf 75 °C und Phase B separat auf 80 °C erwärmt und anschließend Phase B unter Rühren langsam in die Phase A eingetragen. Die vereinigten Phasen werden homogenisiert. Danach wird unter Rühren abgekühlt und bei 35 °C Phase C zugesetzt.

### Bezugsquellen:

(1) Merck KGaA
(2) Th. Goldschmidt AG
(3) Henkel KGaA
(4) BASF AG
(5) H.B. Fuller GmbH
(6) ISP

Die Kombination der Lichtschutzfilter Eusolex 2292 und Eusolex 9020 wirkt der Verfärbung der Emulsion durch Lagerung unter Tageslicht, durch UV-Bestrahlung und durch Lagerung unter Lichtausschluß signifikant entgegen.

### Beispiel 10

Aus folgenden Komponenten wird eine erfindungsgemäße W/O-Emulsion enthaltend Vitamin K1 hergestellt:

| | | | Gew.-% |
|---|---|---|---|
| A Eusolex T-2000 | (Art.-Nr. 1.05373) | (1) | 3,0 |
| Eusolex 4360 | (Art.-Nr. 1.05376) | (1) | 2,0 |
| Isolan PDI | | (2) | 3,0 |
| Cetiol V | | (3) | 8,0 |
| Luvitol EHO | | (4) | 8,5 |
| Lunacera M | | (5) | 0,5 |
| B Glycerin (etwa 87%) | (Art.-Nr. 1.04091) | (1) | 3,0 |
| Magnesiumsulfat | | | |
| Heptahydrat | (Art.-Nr.1.05882) | (1) | 1,0 |
| Euxyl K100 | | (6) | 0,5 |
| Wasser, demineralisiert | | | ad 100 |
| | | | |
| C Vitamin K1 | | (1) | 1,0 |

### Herstellung:

Die Phase A wird auf 75 °C und Phase B separat auf 80 °C erwärmt und anschließend Phase B unter Rühren langsam in die Phase A eingetragen. Die vereinigten Phasen werden homogenisiert. Danach wird unter Rühren abgekühlt und bei 35 °C Phase C zugesetzt.

### Bezugsquellen:

(1) Merck KGaA
(2) Th. Goldschmidt AG
(3) Henkel KGaA
(4) BASF AG
(5) H.B. Fuller GmbH
(6) Schülke & Mayr

Diese Emulsion zeigt eine deutlich geringere Verfärbung im Vergleich zu den in den Beispielen 6-9 untersuchten Emulsionen.

## Patentansprüche

1. Kosmetische oder dermatologische Formulierung enthaltend Hilfs- und/oder Trägerstoffe sowie 2-Hydroxy-5-methyllaurophenonoxim als Wirkstoff, **dadurch gekennzeichnet, daß** zusätzlich
(a) ein oder mehrere Antioxidantien
und/oder
(b) ein oder mehrere UV-Filter
enthalten ist.

2. Kosmetische oder dermatologische Formulierung nach Anspruch 1, **dadurch gekennzeichnet, daß** als Antioxidans Butylhydroxytoluol enthalten ist.

3. Kosmetische oder dermatologische Formulierung nach Anspruch 1, **dadurch gekennzeichnet, daß** als Antioxidans eine oder mehrere Verbindungen ausgewählt aus Flavonoiden und/oder Coumaranonen enthalten ist.

4. Kosmetische oder dermatologische Formulierung nach Anspruch 3, **dadurch gekennzeichnet, daß** die Flavonoide und Coumaranone ausgewählt sind aus den Verbindungen der Formel (I): 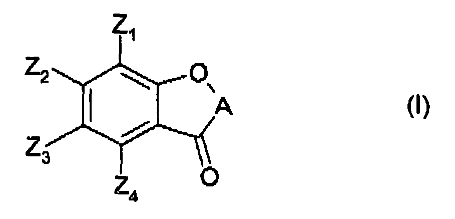 worin
Z₁ bis Z₄ jeweils unabhängig voneinander H, OH, Alkoxy, Hydroxy-alkoxy, Mono- oder Oligoglycosidreste bedeuten und wobei die Alkoxy- und Hydroxyalkoxygruppen verzweigt und unverzweigt sein und 1 bis 18 C-Atome aufweisen können und wobei an die Hydroxygruppen der genannten Reste auch Sulfat oder Phosphat gebunden sein kann,
A ausgewählt ist aus der Gruppe bestehend aus den Teilformeln (IA), (IB) und (IC) 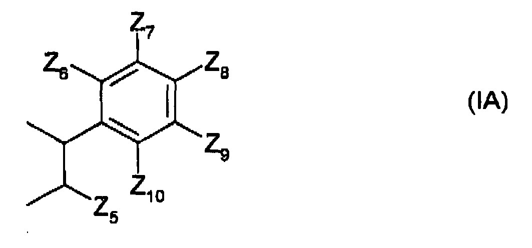 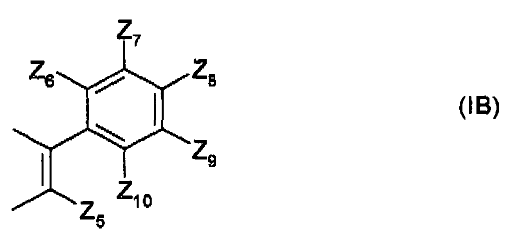 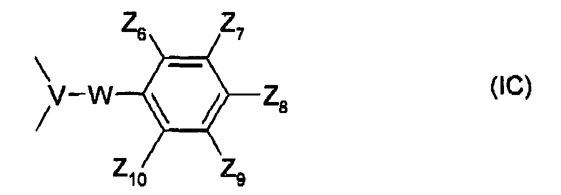
Z₅ H, OH oder OR,
R einen Mono- oder Oligoglykosidrest,
Z₆ bis Z₁₀ die Bedeutung der Reste Z₁ bis Z₄ besitzen, und
bedeutet,

5. Kosmetische oder dermatologische Formulierung nach Anspruch 4, **dadurch gekennzeichnet, daß** das Flavonoid Rutin oder Troxerutin ist.

6. Kosmetische oder dermatologische Formulierung nach einem der Ansprüche 1 bis 5, **dadurch gekennzeichnet, daß** der eine oder die mehreren UV-Filter ausgewählt sind aus folgenden Substanzen: Zinkoxid, Titandioxid, 3-(4'-Methylbenzyliden)-dl-kampfer, 1-(4-tert-Butylphenyl)-3-(4-methoxyphenyl)propan-1,3-dion, 4-lsopropyldibenzoylmethan, 2-Hydroxy-4-methoxybenzophenon, Methoxyzimtsäureoctylester, 3,3,5-Trimethylcyclohexylsalicyfat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihren Kalium-, Natrium- und Triethanolaminsalzen.

7. Kosmetische oder dermatologische Formulierung nach Anspruch 6, **dadurch gekennzeichnet, daß** als UV-Filter Zinkoxid und/oder Titandioxid enthalten ist.

8. Kosmetische oder dermatologische Formulierung nach Anspruch 7, **dadurch gekennzeichnet, daß** als UV-Filter Titandioxid und ein oder mehrere weitere UV-Filter ausgewählt aus 3-(4'-Methylbenzyliden)-di-kampfer, 1-(4-tert-Butylphenyl)-3-(4-methoxyphenyl)propan-1,3-dion, 4-lsopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihren Kalium-, Natrium- und Triethanolaminsalzen enthalten ist.

9. Kosmetische oder dermatologische Formulierung nach Anspruch 8, **dadurch gekennzeichnet, daß** die weiteren UV-Filter ausgewählt sind aus 2-Hydroxy-4-methoxybenzophenon und Methoxyzimtsäureoctylester.

10. Kosmetische oder dermatologische Formulierung nach einem der Ansprüche 1 bis 9, **dadurch gekennzeichnet, daß** der Anteil des Wirkstoffs von 0,001 bis 20 Gew.%, der Anteil der einen oder der mehreren Antioxidantien von 0,001 bis 5 Gew,% und der Anteil der einen oder der mehreren UV-Filter von 0,05 bis 20 Gew.%, jeweils bezogen auf die gesamte Formulierung, ist.

11. Kosmetische oder dermatologische Formulierung nach einem der Ansprüche 1 bis 10, **dadurch gekennzeichnet, daß** sie in Form einer Lösung, einer Suspension, einer Emulsion, einer Paste, einer Salbe, eines Gels, einer Creme, einer Lotion, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- und After-Sun-Präparats oder eines Sprays, eines Sticks, Shampoos oder Duschbads vorliegt.

12. Kosmetische oder dermatologische Formulierung nach Anspruch 11, **dadurch gekennzeichnet, daß** sie in Form einer W/O-Emulsion vorliegt.

13. Verwendung von einer oder mehreren Verbindungen ausgewählt aus Antioxidantien und UV-Filtern zum Schutz und/oder zur Stabilisierung von in einer kosmetischen oder dermatologischen Formulierung enthaltenem Wirkstoff 2-Hydroxy-5-methyllaurophenonoxim.

14. Verwendung von 2-Hydroxy-5-methyllaurophenonoxim und einer oder mehreren Substanzen ausgewählt aus Antioxidantien und/oder UV-Filtern zur Herstellung einer kosmetischen oder dermatologischen Formulierung.

15. Verwendung nach Anspruch 14 zum Schutz oder zur Pflege der menschlichen oder tierischen Haut.

16. Verfahren zur Herstellung einer kosmetischen oder dermatologischen Formulierung, **dadurch gekennzeichnet, daß** man 2-Hydroxy-5-methyllaurophenonoxim und eine oder mehrere Substanzen ausgewählt aus Antioxidantien und UV-Filtern gegebenenfalls mit Hilfs- und/oder Trägerstoffen in eine geeignete kosmetische oder dermatologische Formulierungsform bringt.

## Claims

1. Cosmetic or dermatological formulation comprising assistants and/or vehicles and 2-hydroxy-5-methyllaurophenone oxime as active compound, **characterised in that**
(a) one or more antioxidants
and/or
(b) one or more UV filters
are additionally present.

2. Cosmetic or dermatological formulation according to Claim 1, **characterised in that** the antioxidant present is butylhydroxytoluene.

3. Cosmetic or dermatological formulation according to Claim 1, **characterised in that** the antioxidant present is one or more compounds selected from flavonoids and/or coumaranones.

4. Cosmetic or dermatological formulation according to Claim 3, **characterised in that** the flavonoids and coumaranones are selected from the compounds of the formula (I): 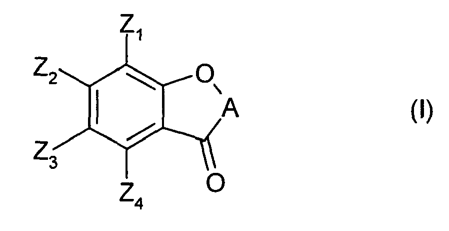 in which
Z₁ to Z₄ each, independently of one another, denote H, OH, alkoxy, hydroxyalkoxy, mono- or oligoglycoside radicals and where the alkoxy and hydroxyalkoxy groups may be branched and unbranched and may have 1 to 18 C atoms and where sulfate or phosphate may also be bonded to the hydroxyl groups of the said radicals,
A is selected from the group consisting of the sub-formulae (IA), (IB) and (IC) 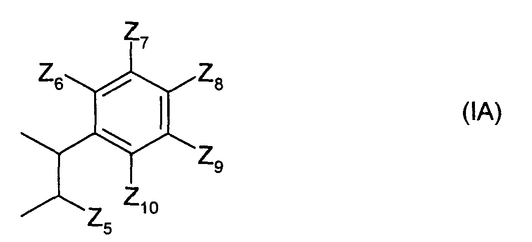 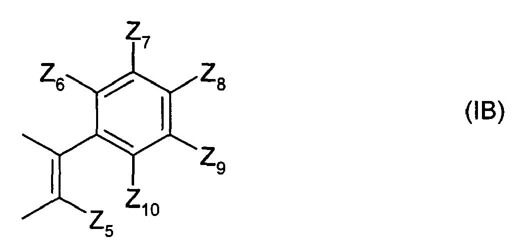 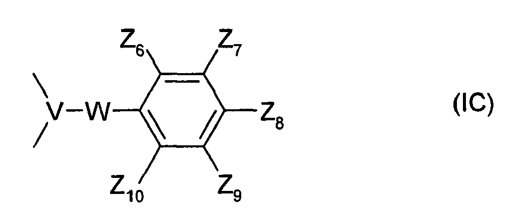
Z₅ denotes H, OH or OR,
R denotes a mono- or oligoglycoside radical,
Z₆ to Z₁₀ have the meaning of the radicals Z₁ to Z₄, and

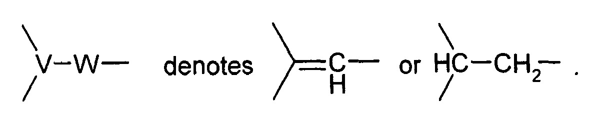

5. Cosmetic or dermatological formulation according to Claim 4, **characterised in that** the flavonoid is rutin or troxerutin.

6. Cosmetic or dermatological formulation according to one of Claims 1 to 5, **characterised in that** the one or more UV filters are selected from the following substances: zinc oxide, titanium dioxide, 3-(4'-methylbenzyllidene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and the potassium, sodium and triethanolamine salts thereof.

7. Cosmetic or dermatological formulation according to Claim 6, **characterised in that** the UV filter present is zinc oxide and/or titanium dioxide.

8. Cosmetic or dermatological formulation according to Claim 7, **characterised in that** the UV filter present is titanium dioxide and one or more further UV filters selected from 3-(4'-methylbenzylidene)-di-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and the potassium, sodium and triethanolamine salts thereof.

9. Cosmetic or dermatological formulation according to Claim 8, **characterised in that** the further UV filters are selected from 2-hydroxy-4-methoxybenzophenone and octyl methoxycinnamate.

10. Cosmetic or dermatological formulation according to one of Claims 1 to 9, **characterised in that** the proportion of the active compound is from 0.001 to 20% by weight, the proportion of the one or more antioxidants is from 0.001 to 5% by weight, and the proportion of the one or more UV filters is 0.05 to 20% by weight, in each case based on the formulation as a whole.

11. Cosmetic or dermatological formulation according to one of Claims 1 to 10, **characterised in that** it is in the form of a solution, a suspension, an emulsion, a paste, an ointment, a gel, a cream, a lotion, a powder, a soap, a surfactant-containing cleansing preparation, an oil, a lipstick, a lip-care stick, a mascara, an eyeliner, eyeshadow, rouge, powder, emulsion or wax make-up, a sunscreen, pre-sun or after-sun preparation or a spray, a stick, shampoo or shower preparation.

12. Cosmetic or dermatological formulation according to Claim 11, **characterised in that** it is in the form of a W/O emulsion.

13. Use of one or more compounds selected from antioxidants and UV filters for the protection and/or stabilisation of the active compound 2-hydroxy-5-methyllaurophenone oxime present in a cosmetic or dermatological formulation.

14. Use of 2-hydroxy-5-methyllaurophenone oxime and one or more substances selected from antioxidants and/or UV filters for the preparation of a cosmetic or dermatological formulation.

15. Use according to Claim 14 for the protection or care of human or animal skin.

16. Process for the preparation of a cosmetic or dermatological formulation, **characterised in that** 2-hydroxy-5-methyllaurophenone oxime and one or more substances selected from antioxidants and UV filters, if desired with assistants and/or vehicles, are converted into a suitable cosmetic or dermatological formulation form.

## Revendications

1. Formulation cosmétique ou dermatologique comprenant des auxiliaires et/ou des véhicules et de la 2-hydroxy-5-méthyllaurophénone oxime comme composé actif, **caractérisée en ce que**
(a) un ou plusieurs antioxydants
et/ou
(b) un ou plusieurs filtres UV
sont également présents.

2. Formulation cosmétique ou dermatologique selon la revendication 1, **caractérisée en ce que** l'antioxydant présent est le butylhydroxy-toluène.

3. Formulation cosmétique ou dermatologique selon la revendication 1, **caractérisée en ce que** l'antioxydant présent est constitué par un ou plusieurs composés sélectionnés parmi les flavonoïdes et/ou les coumaranones.

4. Formulation cosmétique ou dermatologique selon la revendication 3, **caractérisée en ce que** les flavonoïdes et les coumaranones sont sélectionnés parmi les composés de formule (I): 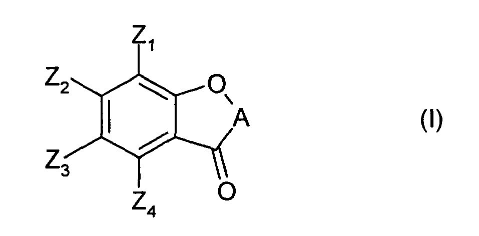 dans laquelle
Z₁ à Z₄ désignent chacun, indépendamment les uns des autres, des radicaux H, OH, alcoxy, hydroxyalcoxy, mono- ou oligoglycoside et où les groupements alcoxy et hydroxy-alcoxy peuvent être ramifiés ou non et peuvent contenir de 1 à 18 atomes de C et où du sulfate ou du phosphate peut également être lié aux groupements hydroxyle desdits radicaux,
A est sélectionné parmi le groupe constitué par les sous-formules (IA), (IB) et (IC) 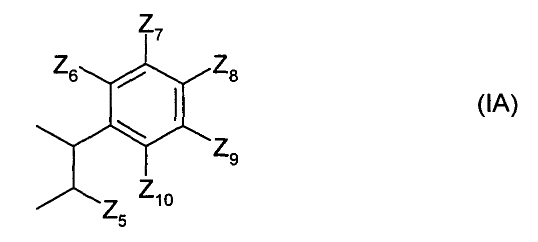 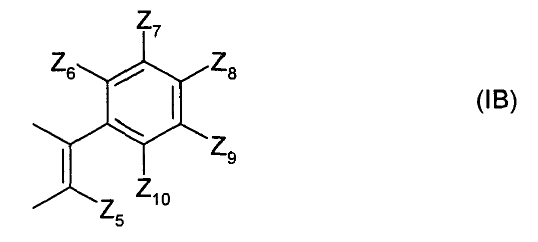 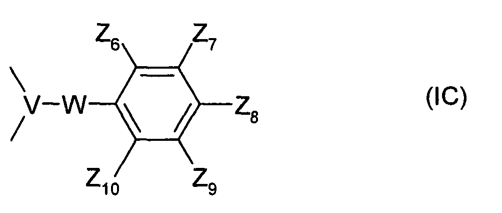
Z₅ désigne H, OH ou OR,
R désigne un radical mono- ou oligoglycoside,
Z₆ à Z₁₀ ont la signification des radicaux Z₁ à Z₄, et

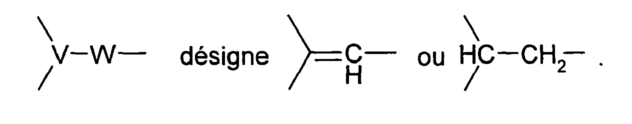

5. Formulation cosmétique ou dermatologique selon la revendication 4, **caractérisée en ce que** le flavonoïde est la rutine ou la troxérutine.

6. Formulation cosmétique ou dermatologique selon l'une des revendications 1 à 5, **caractérisée en ce que** le (les) un ou plusieurs filtres UV est (sont) sélectionné(s) parmi les substances suivantes: l'oxyde de zinc, le dioxyde de titane, le 3-(4'-méthylbenzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenz-imidazole-5-sulfonique et leurs sels de potassium, de sodium et de triéthanolamine.

7. Formulation cosmétique ou dermatologique selon la revendication 6, **caractérisée en ce que** le filtre UV présent est l'oxyde de zinc et/ou le dioxyde de titane.

8. Formulation cosmétique ou dermatologique selon la revendication 7, **caractérisée en ce que** le filtre UV présent est le dioxyde de titane et un ou plusieurs filtres UV supplémentaires sélectionnés parmi le 3-(4'-méthylbenzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthyl-amino)benzoate de 2-éthylhexyle, le 2-cyano-3,3-diphénylacrylate de 2-éthylhexyle, l'acide 2-phénylbenzimidazole-5-sulfonique et leurs sels de potassium, de sodium et de triéthanolamine.

9. Formulation cosmétique ou dermatologique selon la revendication 8, **caractérisée en ce que** les filtres UV supplémentaires sont sélectionnés parmi la 2-hydroxy-4-méthoxybenzophénone et le méthoxycinnamate d'octyle.

10. Formulation cosmétique ou dermatologique selon l'une des revendications 1 à 9, **caractérisée en ce que** la proportion du composé actif va de 0,001 à 20% en poids, la proportion du (des) un ou plusieurs antioxydants va de 0,001 à 5% en poids, et la proportion du (des) un ou plusieurs filtres UV va de 0,05 à 20% en poids, dans chaque cas par rapport à la formulation globale.

11. Formulation cosmétique ou dermatologique selon l'une des revendications 1 à 10, **caractérisée en ce qu'**elle est sous forme d'une solution, d'une suspension, d'une émulsion, d'une pâte, d'un onguent, d'un gel, d'une crème, d'une lotion, d'une poudre, d'un savon, d'une préparation nettoyante contenant des tensioactifs, d'une huile, d'un rouge à lèvres, d'un bâton pour le soin des lèvres, d'un mascara, d'un eye-liner, d'un fard à paupières, d'un fard à joues, d'une poudre, émulsion ou cire de maquillage, d'un filtre solaire, d'une préparation pré-solaire ou après-solaire ou d'un spray, d'un bâton, d'un shampooing ou d'une préparation pour douche.

12. Formulation cosmétique ou dermatologique selon la revendication 11, **caractérisée en ce qu'**elle est sous forme d'une émulsion E/H.

13. Utilisation d'un ou plusieurs composés sélectionnés parmi les antioxydants et les filtres UV pour la protection et/ou la stabilisation du composé actif, le 2-hydroxy-5-méthyllaurophénone oxime, présent dans une formulation cosmétique ou dermatologique.

14. Utilisation du 2-hydroxy-5-méthyllaurophénone oxime et d'une ou plusieurs substances sélectionnées parmi les antioxydants et/ou les filtres UV pour la préparation d'une formulation cosmétique ou dermatologique.

15. Utilisation selon la revendication 14, pour la protection ou le soin de la peau humaine ou animale.

16. Procédé de préparation d'une formulation cosmétique ou dermatologique, **caractérisée en ce que** le 2-hydroxy-5-méthyllaurophénone oxime et une ou plusieurs substances sélectionnées parmi les antioxydants et les filtres UV, si on le souhaite avec des auxiliaires et/ou véhicules, sont convertis en une forme de formulation cosmétique ou dermatologique convenable.
